# EUROPEAN PATENT APPLICATION

(11) **EP 1 783 207 A1**
(43) Date of publication of application: **09.05.2007**
(21) Application number: 05751180.0
(22) Date of filing: 15.06.2005
(51) Int. Cl.: C12N 5/06, A61K 35/12, A61L 27/00

(54) **METHOD OF PRODUCING VASCULAR ENDOTHELIAL CELLS FROM PRIMATE EMBRYONIC STEM CELLS**

(30) Priority: 22.06.2004 JP 2004184138
(71) Applicant: TANABE SEIYAKU CO., LTD., Chuo-ku, Osaka-shi, Osaka 541-8505 (JP)
(72) Inventor: NAKAO, Kazuwa, Nishikyo-ku, Kyoto-shi, Kyoto 610-1101 (JP); ITOH, Hiroshi, Sakyo-ku, Kyoto-shi, Kyoto 606-0835 (JP); YAMASHITA, Jun, Nakagyo-ku, Kyoto-shi, Kyoto 604-0901 (JP); SONE, Masakatsu, Kyoto-shi, Kyoto 606-8357 (JP); YAMAHARA, Kenichi, Otsu-shi, Shiga 520-0113 (JP); KONDO, Yasushi, Chuo-ku, Osaka-shi, Osaka 541-8505 (JP); SUZUKI, Yutaka, Chuo-ku, Osaka-shi, Osaka 541-8505 (JP)
(74) Representative: HOFFMANN EITLE
(86) International application number: PCT/JP2005/010962
(87) International publication number: WO 2005/123904

(57) **Abstract**

To provide a method for differentiating primate embryonic stem cells into vascular endothelial cells, and a technique using the method. A method for differentiating primate embryonic stem cells into early developmental vascular endothelial cells comprising differentiating primate embryonic stem cells into vascular endothelial cell parker-positive cell population; the early developmental vascular endothelial cells; a method for producing the vascular endothelial cells; a method for amplifying the vascular endothelial cells; a vascular lesion-therapeutic agent containing the early developmental vascular endothelial cells as an active ingredient; a method for revascularization comprising supplying the early developmental vascular endothelial cells; a method for transplantation comprising transplanting the early developmental vascular endothelial cells; and a method for treatment comprising administering the early developmental vascular endothelial cells in a therapeutically effective dose.

## Description

### TECHNICAL FIELD

The present invention relates to a method for differentiating primate embryonic stem cells into vascular endothelial cells, and a technique using the method. More specifically, the present invention relates to a method for differentiating primate embryonic stem cells into vascular endothelial cells (for example, early developmental vascular endothelial cells, and the like), a method for producing the vascular endothelial cells (for example, early developmental vascular endothelial cells, and the like), a method for amplifying the early developmental vascular endothelial cells, early developmental vascular endothelial cells obtained by the method for production, a vascular lesion-therapeutic agent, a method for revascularization, use of the early developmental vascular endothelial cells, a method for treating a condition and/or a disease in which a therapeutic effect can be expected by ameliorating local blood flow, a method for constructing a vascular structure, and the like.

### BACKGROUND ART

Recently, studies on the differentiation and regeneration of various organs have been advanced, and the regenerative therapies in the field of clinical medicine have been brought into view as actual subjects of studies. Therefore, it has been placed more expectations on regenerative medicine day by day. Inter alias, a study using embryonic stem cells having an ability to differentiate into all types of cells or organs in a living body is one of main themes in clarification of the mechanism of development or differentiation, and regenerative medicine.

So far, studies on mouse embryonic stem cells have been progressed. In addition, embryonic stem cells of a primate such as a human and a monkey has been established. It has been revealed, however, that primate embryonic stem cells greatly differs from mouse embryonic stem cells in the aspect of, for example, a form of a colony, expression manners of cell surface antigens, a proliferation and division rate, and dependency on leukemia inhibitory factor (LIF) [Non-Patent Publication 1].

On the other hand, in the regenerative therapy for vascular vessel, for example, a clinical trial regarding an angiogenesis medicine using a single angiogenesis factor such as vascular endothelial growth factor (VEGF), basic fibroblast growth factor (bFGF), hepatocyte growth factor (HGF) or the like has been performed on ischemic diseases. However, as described above, the angiogenesis therapy using angiogenesis factor has a defect that the angiogenesis medicine has disadvantages in safety, since according to a random double blind control clinical trial when bFGF is used as an angiogenesis factor for a coronary artery disease, a symptom is likely to be ameliorated by the angiogenesis medicine in a short term but not in a long term.

Non-Patent Publication 1: Pera et al., Journal of Cell Science, 113, 5-10 (2000)

### DISCLOSURE OF INVENTION

### PROBLEMS TO BE SOLVED BY THE INVENTION

The present invention relates to the provision of a means of differentiating primate embryonic stem cells into vascular endothelial cells or early developmental vascular endothelial cells, or a technique using the means.

In a first aspect, the present invention relates to the provision of a method for differentiating primate embryonic stem cells into early developmental vascular endothelial cells, capable of achieving at least any one of the provision of early developmental vascular endothelial cells which achieves at least any one of long-term culture, subculture, *in vitro* proliferation, a graft survival at a high efficiency, obtainment of a high compatibility with an individual, development of vascular endothelial cells in an individual, treatment of a condition and/or a disease in which a therapeutic effect can be expected by ameliorating local blood flow, and the like; the supply of the early developmental vascular endothelial cells in a large amount; the provision of a material for the treatment of a condition and/or a disease in which a therapeutic effect can be expected by ameliorating local blood flow; the evaluation of a factor that exhibits its function in a differentiation process from primate embryonic stem cells to early developmental vascular endothelial cells; and the like.

In a second aspect, the present invention relates to the provision of a method for producing early developmental vascular endothelial cells, capable of achieving at least any one of the provision of early developmental vascular endothelial cells having excellent properties that make it possible to have at least any one of long-term culture, subculture, *in vitro* proliferation, a graft survival at a high efficiency, obtainment of a high compatibility with an individual, development of vascular endothelial cells in an individual, treatment of a condition and/or a disease in which a therapeutic effect can be expected by ameliorating local blood flow, and the like; the supply of the early developmental vascular endothelial cells in a large amount; the provision of a material for the treatment of a condition and/or a disease in which a therapeutic effect can be expected by ameliorating local blood flow; and the like.

In addition, in a third aspect, the present invention relates to the provision of a method for amplifying early developmental vascular endothelial cells which achieves at least any one of the supply of the early developmental vascular endothelial cells in a large amount, the obtainment of early developmental vascular endothelial cells having a substantially uniform quality, and the like.

Further, in a fourth aspect, the present invention relates to the provision of a vascular lesion-therapeutic agent, which achieves at least any one of an efficient treatment of a vascular lesion, treatment of a condition and/or a disease in which a therapeutic effect can be expected by ameliorating local blood flow, and the like.

In a fifth aspect, the present invention relates to the provision of early developmental vascular endothelial cells which achieves at least any one of long-term culture, subculture, *in vitro* proliferation, a graft survival at a high efficiency, obtainment of a high compatibility with an individual, development of vascular endothelial cells in an individual, treatment of a condition and/or a disease in which a therapeutic effect can be expected by ameliorating local blood flow, and the like.

In addition, in a sixth aspect, the present invention relates to the provision of a method for revascularization, which achieves at least any one of treatment of a vascular lesion site, treatment of a condition and/or a disease in which a therapeutic effect can be expected by ameliorating local blood flow, regeneration of a blood vessel in a site in need of regeneration of a blood vessel, and the like.

In a seventh aspect, the present invention relates to the provision of a method for transplantation, which achieves at least any one of obtainment of a graft survival at a high efficiency, obtainment of a high compatibility with an individual, development of a functional blood vessel that communicates with a blood vessel of an individual *per se* from the transplanted early developmental vascular endothelial cells, and the like.

In a eighth aspect, the present invention relates to the provision of a method for treating a vascular lesion, which makes it possible to perform treatment of a condition and/or a disease, such as ulcer or an ischemic disease, in which a therapeutic effect can be expected by ameliorating local blood flow.

In a ninth aspect, the present invention relates to the provision of use of the early developmental vascular endothelial cells obtained by the above method for production in the manufacture of a medicament which makes it possible to perform treatment of a condition and/or a disease, in which a therapeutic effect can be expected by ameliorating local blood flow, and the like.

In a tenth aspect, the present invention relates to the provision of a method for constructing a vascular structure, which makes it possible to achieve at least any one of the provision of a material for the treatment of a condition and/or a disease in which a therapeutic effect can be expected by ameliorating local blood flow, capable of achieving at least any one of construction of a vascular structure, obtainment of a vascular structure showing a high compatibility with an individual, obtainment of a vascular structure capable of having a graft survival at a high efficiency, and the like.

Further objects of the present invention will be apparent from the following description.

### MEANS TO SOLVE THE PROBLEMS

Specifically, the gist of the present invention relates to:
(1) a method for differentiating primate embryonic stem cells into early developmental vascular endothelial cells, characterized in that the method comprises differentiating primate embryonic stem cells into vascular endothelial cell marker-positive cell population;
(2) the method according to the above (1), wherein the method comprises the steps of:
   (I) co-culturing primate embryonic stem cells and feeder cells, thereby differentiating into a cell population A containing vascular endothelial cell marker-positive cells; and
   (II) substantially separating the vascular endothelial cell marker-positive cells from the cell population A obtained in the above step (I);
(3) a method for producing early developmental vascular endothelial cells, comprising the steps of:
   (I) co-culturing primate embryonic stem cells and feeder cells, thereby differentiating into a cell population A containing vascular endothelial cell marker-positive cells; and
   (II) substantially separating the vascular endothelial cell marker-positive cells from the cell population A obtained in the above step (I);
(4) the method according to the above (3), further comprising the step of (III) amplifying the cells obtained in the above step (II);
(5) a method for amplifying early developmental vascular endothelial cells, comprising the steps of:
   (I) co-culturing primate embryonic stem cells and feeder cells, thereby differentiating into a cell population A containing vascular endothelial cell marker-positive cells;
   (II) substantially separating the vascular endothelial cell marker-positive cells from the cell population A obtained in the above step (I); and
   (III) amplifying the cells obtained in the above step (II);
(6) early developmental vascular endothelial cells, obtainable by the method as defined in the above (3) or (4);
(7) a vascular lesion-therapeutic agent, comprising early developmental vascular endothelial cells obtainable by differentiating primate embryonic stem cells as an active ingredient;
(8) a method for revascularization, characterized in that the method comprises supplying early developmental vascular endothelial cells obtainable by differentiating primate embryonic stem cells to a site in need of regeneration of a blood vessel;
(9) a method for revascularization, characterized in that the method comprises supplying early developmental vascular endothelial cells obtainable by the method as defined in the above (3) or (4) to a site in need of regeneration of a blood vessel;
(10) a method for transplantation, characterized in that the method comprises transplanting early developmental vascular endothelial cells obtainable by the method as defined in the above (3) or (4) into a living body;
(11) a method for treating a condition or a disease in which a therapeutic effect can be expected by ameliorating local blood flow in a mammal individual to be tested, characterized in that the method comprises administering early developmental vascular endothelial cells obtainable by the method as defined in the above (3) or (4) in a therapeutically effective dose to the mammal individual to be tested, wherein the mammal individual to be tested has a condition or a disease in which a therapeutic effect can be expected by ameliorating local blood flow;
(12) use of early developmental vascular endothelial cells obtainable by differentiating primate embryonic stem cells in the manufacture of a medicament for treating a mammal individual to be tested by supplying to a site in need of regeneration of a blood vessel in the mammal individual to be tested, and revascularizing the site, the mammal individual having a condition and/or a disease, in which a therapeutic effect can be expected by ameliorating local blood flow;
(13) a method for constructing a vascular structure, characterized in that the method comprises culturing early developmental vascular endothelial cells obtainable by differentiating primate embryonic stem cells in an environment appropriate for angiogenesis;
(14) the method according to the above (13), comprising the steps of:
   (I) co-culturing primate embryonic stem cells and feeder cells, thereby differentiating into a cell population A containing vascular endothelial cell marker-positive cells;
   (II) substantially separating the vascular endothelial cell marker-positive cells from the cell population A obtained in the above step (I); and
   (III') culturing the above vascular endothelial cell marker-positive cells in an environment appropriate for angiogenesis, or a blood vessel environment; and
(15) the method according to above (14), further comprising the step, prior to the step (III'), of (III) amplifying the cells obtained in the above step (II).

### EFFECTS OF THE INVENTION

According to the method for differentiating primate embryonic stem cells into early developmental vascular endothelial cells of the present invention, there are exhibited some excellent effects that the early developmental vascular endothelial cells having excellent characteristics in that long-term culture, subculture, and *in vitro* proliferation can be performed, that a graft survival at a high efficiency or a high compatibility with an individual is shown, vascular endothelial cells can be developed in an individual, and that a condition and/or a disease in which a therapeutic effect can be expected by ameliorating local blood flow, and the like can be treated, can be supplied in a large amount. In addition, according to the method for differentiation of the present invention, an excellent effect that a material for the treatment of a condition and/or a disease in which a therapeutic effect can be expected by ameliorating local blood flow can be provided, is exhibited. Further, an excellent effect that a factor that exhibits its function in a differentiation process from primate embryonic stem cells to early developmental vascular endothelial cells can be evaluated is exhibited.

According to the method for producing early developmental vascular endothelial cells of the present invention, an excellent effect that early developmental vascular endothelial cells having the excellent properties mentioned above can be supplied in a large amount is exhibited. Also, according to the method for production of the present invention, an excellent effect that a material for the treatment of a condition and/or a disease in which a therapeutic effect can be expected by ameliorating local blood flow, and the like can be stably provided in a large amount is exhibited.

Further, according to the method for amplifying early developmental vascular endothelial cells of the present invention, an excellent effect that early developmental vascular endothelial cells having a substantially uniform quality can be obtained in a large amount is exhibited.

According to the early developmental vascular endothelial cells of the present invention, there are exhibited some excellent effects that long-term culture and subculture can be performed, and that *in vitro* proliferation can be performed. Also, according to the early developmental vascular endothelial cells of the present invention, an excellent effect that the cells can be subjected to a graft survival at a high efficiency is exhibited. According to the early developmental vascular endothelial cells of the present invention, there are exhibited some excellent effects that the cells can be used in an individual with a high compatibility, that vascular endothelial cells can be developed in an individual, and that the cells enable the treatment of a condition and/or a disease in which a therapeutic effect can be expected by ameliorating local blood flow.

In addition, according to the vascular lesion-therapeutic agent of the present invention, an excellent effect that a vascular lesion can be efficiently treated is exhibited. Further, according to the vascular lesion-therapeutic agent of the present invention, an excellent effect that a condition and/or a disease in which a therapeutic effect can be expected by ameliorating local blood flow, and the like can be treated is exhibited.

According to the method for revascularization of the present invention, there are exhibited some excellent effects that a vascular lesion site is treated, that a condition and/or a disease in which a therapeutic effect can be expected by ameliorating local blood flow can be treated, and a blood vessel in a site in need of regeneration of a blood vessel can be regenerated. In addition, according to the method for transplantation of the present invention, there are exhibited some excellent effects that a functional blood vessel that communicates with a blood vessel of an individual *per se* from the transplanted early developmental vascular endothelial cells can be developed at a high efficiency and a high compatibility with an individual, and that the method enables treatment of a condition and/or a disease in which a therapeutic effect can be expected by ameliorating local blood flow.

According to use of the early developmental vascular endothelial cells of the present invention, there is exhibited an excellent effect that a medicament enables the treatment of a condition and/or a disease, in which a therapeutic effect can be expected by ameliorating local blood flow, or the like can be produced.

According to the method for treating a vascular lesion of the present invention, an excellent effect that the method enables the treatment of a condition and/or a disease, for example, ulcer, an ischemic disease, arteriosclerosis obliterans, or the like, in which a therapeutic effect can be expected by ameliorating local blood flow is exhibited.

According to the method for constructing a vascular structure of the present invention, an excellent effect that a vascular structure showing a graft survival at a high efficiency and a high compatibility with an individual can be constructed is exhibited. According to the method for constructing a vascular structure of the present invention, an excellent effect that a material for the treatment of a condition and/or a disease in which a therapeutic effect can be expected by ameliorating local blood flow can be provided is exhibited.

### BRIEF DESCRIPTION OF THE DRAWINGS

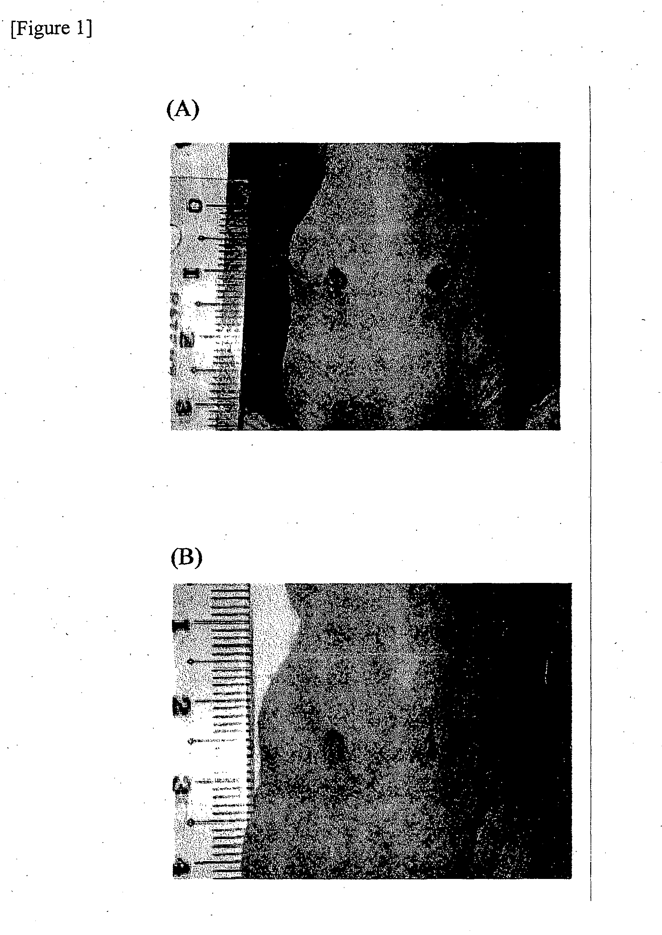

[Figure 1] Figure 1 is an illustration showing a change in external appearance of a skin ulcer site in a skin ulcer model mouse, wherein Panel (A) shows a change in external appearance three days after the transplantation of VE cadherin-positive cells (human early-developmental vascular endothelial cells), and Panel (B) shows gross findings on a sixth day. In each panel, the skin ulcer site on the left side is a site into which VE cadherin-positive cells (early-developmental vascular endothelial cells) are transplanted, and the skin ulcer site on the right side is a site into which phosphate buffered physiological saline is injected (untreated) without transplanting VE cadherin-positive cells thereinto.

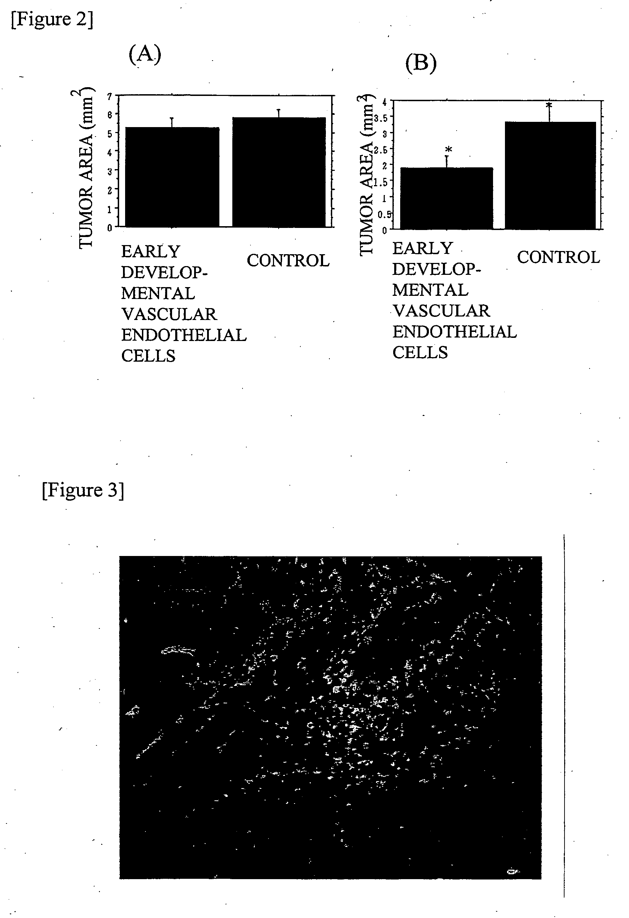

[Figure 2] Figure 2 is a graph showing the area of skin ulcer after the transplantation of VE cadherin-positive cells (human early-developmental vascular endothelial cells) in a skin ulcer model mouse, wherein Panel (A) shows the comparison of the area of skin ulcer three days after the transplantation, and Panel (B) shows the comparison of skin ulcer area six days after the transplantation. In the figures, "VPC" is the area of skin ulcer on the side into which VE cadherin-positive cells are transplanted, and "PBS" is the area of skin ulcer on the side into which phosphate buffered physiological saline is injected (untreated). Here, in Panel (B), p satisfies the condition of p < 0.05. In addition, the results shown in these figures are the examination results for eleven cases.
[Figure 3] Figure 3 shows a tissue section of skin at the site of cell transplantation in a skin ulcer model mouse into which VE cadherin-positive cells (human early-developmental vascular endothelial cells) are transplanted, wherein red color shows DiI-labeled transplanted cells (VE cadherin-positive cells), and green color shows ISOLECTIN B4 (trade name)-labeled (stained) vascular endothelial cells existing in a blood vessel of a KSN nude mouse. In the figure, yellow color shows the portion where the transplanted VE cadherin-positive cells (red) and the vessel of the KSN nude mouse (green) are assessed to be communicated with each other. Scale bar represents 100 µm.

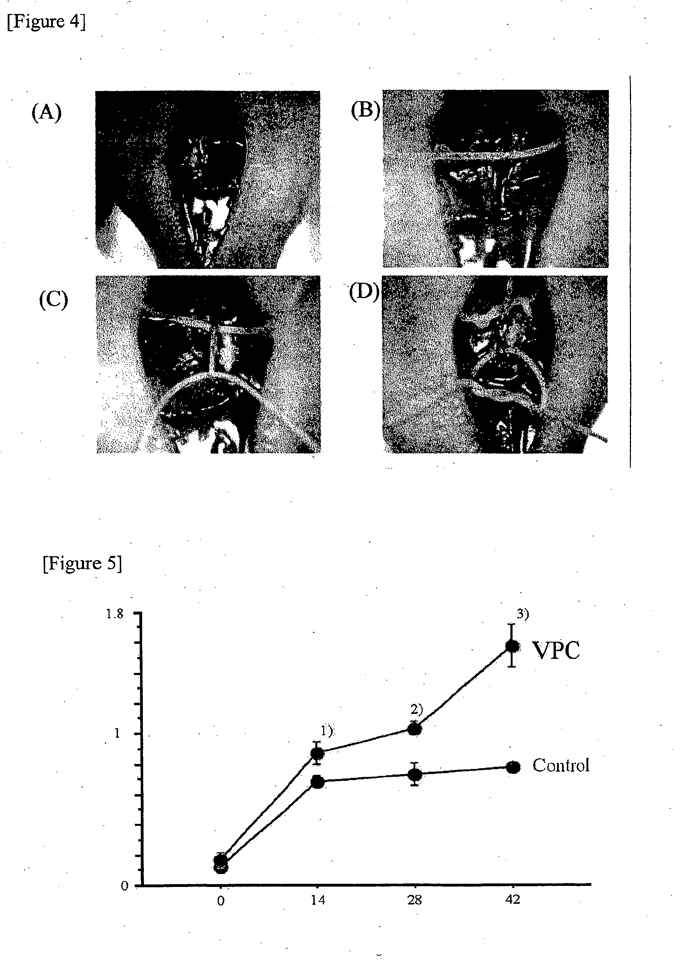

[Figure 4] Figure 4 is an illustration schematically showing transplantation of VE cadherin-positive cells into a femoral artery of a KSN nude mouse, wherein Panel (A) shows the portion where the right femoral region of a KSN nude mouse is vertically incised to expose femoral artery and vein; Panels (B) and (C) show the process of exposing a femoral artery in the portion shown in the above Panel (A); and Panel (D) shows the process of injecting cells into the femoral artery.
[Figure 5] Figure 5 is a graph showing the results of measurement of an effect of ameliorating blood flow by the transplantation of VE cadherin-positive cells (human early-developmental vascular endothelial cells) with a laser Doppler blood flowmeter. In Figure 5, in 1) p satisfies the condition of p<0.05, in 2) p satisfies the condition of p<0.01 and in 3) p satisfies the condition of p<0.001.

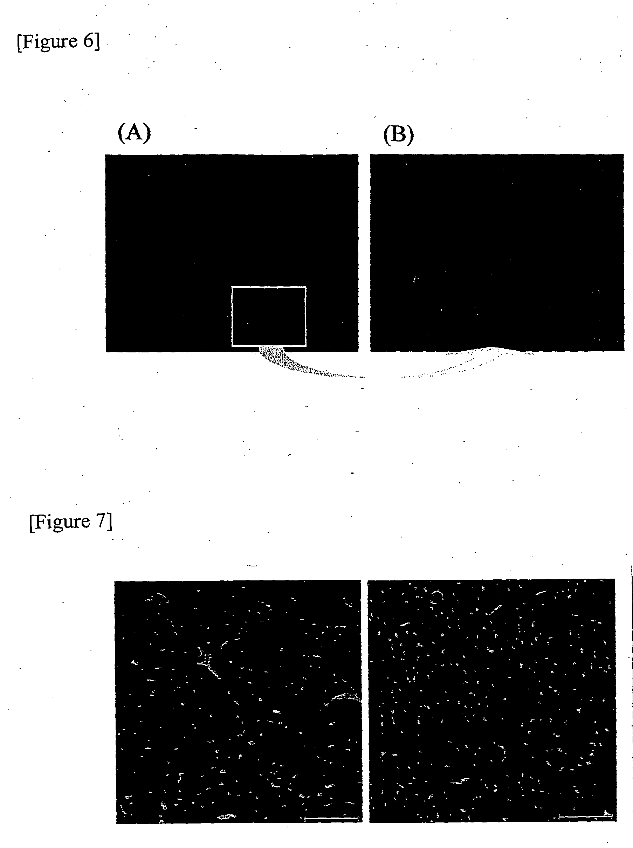

[Figure 6] Figure 6 is an illustration showing the results of examination on a graft survival of transplanted cells in ischemic lateral crural muscle seven days after the transplantation of VE cadherin-positive cells (human early-developmental vascular endothelial cells), wherein red color shows DiI-labeled transplanted cells (VE cadherin-positive cells), and green color shows ISOLECTIN B4 (trade name)-labeled (stained) vascular endothelial cells existing in a blood vessel of a KSN nude mouse. In the figure, yellow color shows the portion where the transplanted VE cadherin-positive cells (red) are assessed to be incorporated into the blood vessel of the KSN nude mouse (green). In Panel (A), a side of the width of the circumscribed portion is 1 mm. In addition, Panel (B) is an enlarged view of the entire circumscribed portion of Panel (A).
[Figure 7] Figure 7 is an illustration showing the results of immunohistological analysis of ischemic lateral crural muscle 42 days after the transplantation of VE cadherin-positive cells (human early-developmental vascular endothelial cells), wherein Panel (A) shows a tissue of a KSN nude mouse (control group), into which phosphate buffered physiological saline is injected without transplanting VE cadherin-positive cells (untreated), and Panel (B) shows a tissue of a KSN nude mouse into which VE cadherin-positive cells are transplanted. In the figure, green color shows mouse PECAM 1, and red color shows human PECAM 1. Each scale bar represents 100 µm.

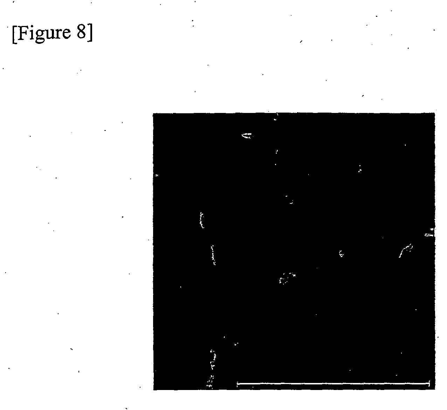

[Figure 8] Figure 8 is an illustration showing the results of examination on incorporation of VE cadherin-positive cells into the blood vessel in ischemic lateral crural muscle seven days after the transplantation of VE cadherin-positive cells (human early-developmental vascular endothelial cells), wherein blue color shows human PECAM 1, and green color shows ISOLECTIN B4 (trade name)-labeled (stained) site. Scale bar represents 100 µm.

### BEST MODE FOR CARRYING OUT THE INVENTION

The present invention is based on the findings of the present inventors that cells obtained by differentiating primate embryonic stem cells into VE-cadherin-positive cells excellently have a grafted survival when transplanted to a living body as early developmental vascular endothelial cells, thereby constructing a functional blood vessel.

One aspect of the present invention relates to a method for differentiating primate embryonic stem cells into early developmental vascular endothelial cells, characterized in that the method comprises differentiating primate embryonic stem cells into vascular endothelial cell marker-positive cell population.

The early developmental vascular endothelial cells obtainable by the method for differentiation of the present invention has excellent characteristics that long-term culture and subculture can be performed, and that *in vitro* proliferation can be performed. In addition, since the early developmental vascular endothelial cells obtainable by the method for differentiation of the present invention are obtained by the differentiation of embryonic stem cells of a desired animal species, for example, human, a monkey, or the like, the cells show some excellent characteristics that the cells shows a graft survival at a high efficiency to a living body, and a high compatibility with an individual. Further, the early developmental vascular endothelial cells obtainable by the method for differentiation of the present invention have some excellent characteristics that vascular endothelial cells can be developed in an individual. Therefore, according to the cells obtained by the method for differentiation of the present invention, the cells enable the treatment of a condition and/or a disease in which a therapeutic effect can be expected by ameliorating local blood flow, or the like.

In the method for differentiation of the present invention, since primate embryonic stem cells that can be unlimitedly amplified by self-replication, especially human embryonic stem cells, are used, according to the method for differentiation of the present invention, early developmental vascular endothelial cells having a substantially uniform quality can be obtained in a large amount. Therefore, according to the method for differentiation of the present invention, a blood vessel constructing material suitable in the utilization in the field of regeneration therapy, a material for the treatment of a condition and/or a disease in which a therapeutic effect can be expected by ameliorating local blood flow, or the like can be efficiently and stably supplied in a large amount.

Further, according to the method for differentiation of the present invention, since primate embryonic stem cells are used, early developmental vascular endothelial cells capable of performing a graft survival in a high efficiency for a living body of a primate, for example, human and monkey, and preferably human, and obtaining a high compatibility.

The phrase "a condition and/or a disease in which a therapeutic effect can be expected by ameliorating local blood flow" mentioned above as used herein includes, for example, ulcer, arteriosclerosis obliterans, an ischemic heart disease, a cerebrovascular disease, Buerger disease, skin ulcer, and the like.

The term "vascular endothelial cells" as used herein means an idea encompassing early developmental vascular endothelial cells and mature vascular endothelial cells, unless specified otherwise.

The term "early developmental endothelial cells" mentioned above as used herein refers to VE-cadherin (Vascular Endothelial-cadherin)-positive and CD34-positive and VEGF (Vascular Endothelial Growth Factor)-R2-positive and PECAM (Platelet and Endothelial Cell Adhesion Molecule) 1-positive cells, each of the cells being differentiated from primate embryonic stem cells. In addition, the term "early developmental vascular endothelial cells" mentioned above has the characteristics such as construction of a functional blood vessel in a living body, to ameliorate blood flow when transplanted to a living body, thereby being distinguishable from the perfectly matured vascular endothelial cells of an adult.

The term "mature vascular endothelial cells" mentioned above as used herein refers to cells showing characteristics equivalent to the perfectly matured vascular endothelial cells of an adult.

The term "primate" as used herein refers to human, a monkey, or the like. The monkey mentioned above is not particularly limited, and includes, for example, a cynomolgus monkey (*Macaca fascicularis*), a rhesus monkey (*Macaca mulatta*), a Japanese monkey (*Macaca fuscata*), a marmoset (*Callithrix jacchus*), and the like.

In addition, the term "primate embryonic stem cells" as used herein refers to undifferentiated cells having both pluripotency and self-renewality. The primate embryonic stem cells mentioned above more concretely refer to cells showing characteristics of alkaline phosphatase (ALP) activity-positive, SSEA (Stage Specific Embryonic Antigen)-3-positive, SSEA-4-positive, TRA (Tumor Rejection Antigen)1-60-positive and TRA1-81-positive. The characteristics mentioned above can be confirmed by a method such as immunostaining with a conventional antibody such as an anti-SSEA-1 antibody, an anti-SSEA-3 antibody, an anti-SSEA-4 antibody, an anti-TRA1-60 antibody or an anti-TRA1-81 antibody, or a method for detecting an alkaline phosphatase using an alkaline phosphatase activity assaying means {for example, commercially available ALP test kit (manufactured by SIGMA) or the like}.

The immunostaining mentioned above can be performed by the conventional procedures {see, for example, Hirashima et al., Blood, 93, 1253-1263 (1999)}.

The term "undifferentiated primate embryonic stem cell marker" as used herein includes, for example, ALP, SSEA-3, SSEA-4, TRA1-60, TRA1-81 and the like.

The term "vascular endothelial marker" usable in the present invention includes those that are usually used in the field of art. Concretely, the vascular endothelial marker includes, for example, VE-cadherin, CD34, PECAM1, and the like. Among them, VE-cadherin is preferable.

The primate embryonic stem cells usable in the present invention includes, for example, CMK-6 cell strain {Hirofumi Suemori et al., Developmental Dynamics, 222, 273-279 (2001)}, HES-3 cell strain {Benjamin E. Reubinof et al., Nature Biotechnology, 18, 399-404 (2000)} and the like. The primate embryonic stem cells mentioned above can be maintained in an undifferentiated state in accordance with the publication by Hirofumi Suemori et al. mentioned above, the publication by Benjamin E. Reubinof et al. mentioned above, or the like.

Here, the primate embryonic stem cells mentioned above can be maintained, proliferated or supplied by culturing the cells in a medium for embryonic stem cells using appropriate feeder cells.

The feeder cells usable for maintaining the undifferentiated state of the embryonic stem cells mentioned above and proliferating the embryonic stem cells may be cells which are conventionally used in culturing embryonic stem cells may be used. For example, the feeder cells mentioned above include cells obtained by subjecting primary culture cells of a fibroblast strain derived from a mouse fetus of the twelfth to sixteenth days of pregnancy, STO cells, which are a mouse fetal fibroblast strain to a treatment with mitomycin C or X-rays.

In addition, the feeder cells usable for maintaining the undifferentiated state of embryonic stem cells and proliferating the embryonic stem cells mentioned above vary depending on the kinds of the cells. For example, when mouse fetus-derived fibroblasts are used as feeder cells, a feeder layer can be obtained by subjecting the mouse fetus-derived fibroblasts to mitomycin C treatment or X-ray irradiation, and culturing the fibroblasts to confluent on a 10 cm dish coated with a 0.1 % by weight aqueous gelatin solution [gelatin derived from cow skin, manufactured by SIGMA, or the like].

The culture conditions for the feeder cells mentioned above vary depending on the kinds of the cells. The conditions include, for example, maintaining at 36° to 38°C, preferably at 37°C in a vapor phase of 5% by volume CO₂ or the like.

In addition, a medium used for maintaining the undifferentiated state of the embryonic stem cells mentioned above and proliferating the embryonic stem cells is not particularly limited. For example, conventional culture medium ingredients depending on an animal species which is a source for supplying embryonic stem cells to be used may be used. Culture medium ingredients vary depending on the kinds of the embryonic stem cells. When used in monkey embryonic stem cells, the culture medium ingredients include, for example, Dulbecco's modified Eagle medium/F12 medium containing β-mercaptoethanol, a non-essential amino acid and serum or a serum substitute (for example, 20% by weight KNOCKOUT-^{™} Serum Replacement) {manufactured by Invitrogen}, and the like. Further, when used in human embryonic stem cells, the culture medium ingredients mentioned above include Dulbecco's modified Eagle medium containing, for example, β-mercaptoethanol, a non-essential amino acid and serum or a serum substitute (for example, 20% by weight KNOCKOUT-^{™} Serum Replacement, manufactured by Invitrogen).

The culture conditions for maintaining the undifferentiated state of the primate embryonic stem cells mentioned above and proliferating the primate embryonic stem cells vary depending on the kinds of embryonic stem cells. The culture conditions include, for example, maintaining at 36° to 38°C, and more preferably at 37°C, preferably in a vapor phase of 5% by volume of CO₂ in the case of the monkey embryonic stem cells, or maintaining at 36° to 38°C, and more preferably at 37°C, preferably in a vapor phase of 5% by volume of CO₂ in the case of the human embryonic stem cells. Here, the culture conditions mentioned above can be set by properly varying the conditions within the range in which the cells can survive and proliferate.

In the case of monkey embryonic stem cells, it is desired that the primate embryonic stem cells mentioned above are cells which are treated by an appropriate means, for example, a reagent suitable for dissociating the cells [for example, trade name: Dissociation Buffer (manufactured by GIBCO), collagenase, dispase, trypsin and the like] and dissociated. In addition, in the case of human embryonic stem cells, when the growth rate is markedly reduced when the cells are separated into single cells, a small cluster of cells obtained by treatment with an appropriate means, for example, a reagent suitable for dissociating cells [for example, collagenase, dispase and the like] may be substituted.

From the viewpoint of possessing an ability to differentiate into vascular endothelial cells and securing a graft survival of cells, the feeder cells mentioned above may be cells that allows promotion of the differentiation from embryonic stem cells to vascular or hemocyte cells that expresses various physiologically active substances or adhesion factors. The feeder cells mentioned above include stromal cells such as calvarial fibroblasts. Concretely, the feeder cells include OP9 cell strain, and the like.

In the method for differentiation of the present invention, the primate embryonic stem cells can be differentiated into a vascular endothelial cell marker-positive cell population by, for example, co-culturing the primate embryonic stem cells and the feeder cells under the conditions appropriate for differentiation.

One of the embodiments of the method for differentiation of the present invention includes a method comprising the steps of:
(I) co-culturing primate embryonic stem cells and feeder cells, thereby differentiating into a vascular endothelial cell marker-positive cell population A; and
(II) substantially separating the vascular endothelial cell marker-positive cells from the cell population A obtained in the above step (I).

In the above step (I), the co-culture of the primate embryonic stem cells and the feeder cells can be performed by, for example, sowing primate embryonic stem cells on the feeder layer mentioned above, and culturing the cells under the conditions suitable for the differentiation.

In the above step (I), it is desired that the amount of the primate embryonic stem cells to be sown on the feeder layer is, for example, preferably 2 × 10⁴ cells to 5 × 10⁵ cells per a 10 cm-diameter cell tissue culture dish, in the case of monkey embryonic stem cells. In the case of human embryonic stem cells, when the cells are sown as a small cluster, the number of cells cannot be counted, so that it may be difficult to accurately grasp the number of cells in some cases. It is desired that the small cluster of the cells are sown at an equivalent cell density to a usual subculture of the embryonic stem cells.

In addition, the culture conditions upon the differentiation vary depending on the kinds of embryonic stem cells. For example, in the case of monkey embryonic stem cells or human embryonic stem cells, the conditions include maintaining at 36° to 38°C, and more preferably at 37°C, preferably for 8 to 10 days, and more preferably for 10 days, preferably in a vapor phase of 5% by volume of CO₂.

The medium usable in the co-culture can be appropriately selected depending upon the kinds of embryonic stem cells, which may be a medium suitable for the differentiation of the embryonic stem cells. The medium includes, for example, a differentiation culture medium such as αMEM containing 5 × 10⁻⁵ M 2-mercaptoethanol and 10% by weight serum.

A culture container usable upon the co-culture may be a container suitable for cell culture. The container includes, for example, a 6 cm-diameter tissue culture dish, a 10 cm-diameter tissue culture dish and the like.

In the above step (I), the concentration of the serum in the medium mentioned above can be appropriately set, and it is desired that the concentration is 10% by weight or so. Here, a functionally equivalent substance, for example, a serum substitute or the like may be used in place of the serum mentioned above.

In the above step (I), the vascular endothelial cell marker-positive cell population A can be confirmed, for example, by a means using an antibody against the vascular endothelial marker mentioned above, for example, immunostaining, flow cytometry or the like, in which the containment of the vascular endothelial cell marker-positive cells is used as an index.

The antibody mentioned above may be any of commercially available antibodies, antibodies prepared by conventional techniques, and their antibody fragments.

Next, the vascular endothelial cell marker-positive cells are substantially separated from the cell population A obtained in the above step (I) {step (II)}.

In the above step (II), the separation of the vascular endothelial cell marker-positive cells mentioned above can be performed by an appropriate cell sorting means, for example, cell sorting by conventional flow cytometry {FACS (Fluorescence Activated Cell Sorter)}; cell sorting by MACS (Magnetic Activated Cell Sorter), or the like. In the FACS mentioned above, the desired cells may be sorted using as an index a label of a labeled antibody suitable for sorting. In addition, in the MACS mentioned above, the desired cells may be sorted by binding magnetic beads retaining an antibody suitable for sorting with the desired cells, and collecting magnetic beads.

The antibody usable upon the cell sorting mentioned above includes at least one kind of an antibody against the vascular endothelial cell marker.

For example, when flow cytometry is used, the desired cells can be collected by suspending a cell population in an appropriate solution, to obtain a cell suspension having an appropriate cell concentration, and applying the resulting cell suspension to a flow cytometer to perform cell sorting.

The term "appropriate solution" usable in suspending the cells mentioned above may be a solution suitable for performing flow cytometry and cell sorting. The appropriate solution includes a phosphate buffered physiological saline (PBS), Hanks' Balanced Salt Solution (HBSS) and the like.

In addition, the cell concentration in the cell suspension mentioned above may be a concentration at which a plurality of cells can be sufficiently discriminated electrically upon flow cytometry and cell sorting, and can be appropriately set depending on the cell sorting instrument to be used. For example, it is desired that the cell concentration in the cell suspension mentioned above is preferably from 1 × 10⁶ cells/ml to 1 × 10⁷ cells/ml.

The conditions for flow cytometry and cell sorting can be appropriately set depending on the sorting systems (for example, water droplet charging system, cell capturing system) in a usable flow cytometer.

The above conditions may be, for example, the number of sorting droplets, the number of cells to be sorted and the flow rate, which are suitable for obtaining the desired cells at a high purity.

When the separation is performed by FACS, a labeling substance used in a labeled antibody includes a fluorescent dye, and the substance can be appropriately selected depending on the kinds of laser beam and a filter of a flow cytometer to be used. The fluorescent dye mentioned above includes, for example, fluorescein isothiocyanate (FITC), phycoerythrin (PE), allophycocyanin (APC), Texas Red (TR), Cy3, Cy5, PerCO (registered trade mark) (manufactured by BD Biosciences), Red613 (registered trade mark) (manufactured by GIBCO), Red670 (registered trade mark) (manufactured by GIBCO), Alexa647 (manufactured by Molecular Sieves), Alexa488 (manufactured by Molecular Sieves) and the like.

Here, upon the cell sorting, when two or more kinds of antibodies are used, each of the antibodies mentioned above may be labeled with a label substance different from each other.

According to the method for differentiation of the present invention, a factor that exhibits its function in a differentiation process from primate embryonic stem cells to early developmental vascular endothelial cells can also be evaluated. Therefore, the method for differentiation of the present invention can be applied to a screening method comprising screening an inducing agent or a blocking agent for differentiation into the early developmental vascular endothelial cells using as an index promotion or blocking of the progress of the differentiation process from primate embryonic stem cells to early developmental vascular endothelial cells on the basis of the method for differentiation with the substances to be evaluated. Here, the screening method can also be applied to the evaluation of a drug.

The substance to be evaluated mentioned above includes low molecular compounds, polypeptides, saccharides, other macromolecular compounds, and the like. In the case where the inducing agent or blocking agent mentioned above is screened, the step (I) may be carried out by adding a substance of the substance to be evaluated to a medium usable in the step (I) of the method for differentiation mentioned above. The promotion and blocking of the progress of the differentiation process is evaluated by using as an index, for example, the number of differentiated cells in a given period of time, the shape of the differentiated cells, the characteristics of the differentiated cells (for example, expression of marker), or the like. The inducing agent mentioned above is useful in the development of a means of treating a condition and/or a disease in which a therapeutic effect can be expected by ameliorating local blood flow. In addition, the blocking agent mentioned above is useful in the development of a therapeutic means on the basis of blocking revascularization in proliferation, metastasis, or the like of a tumor.

Further, according to the method for differentiation of the present invention, since the differentiation can be performed from primate embryonic stem cells to early developmental vascular endothelial cells, the method of the present invention can be applied to the production of the early developmental vascular endothelial cells.

Therefore, another aspect of the present invention relates to a method for producing early developmental vascular endothelial cells, comprising the steps of:
(I) co-culturing primate embryonic stem cells and feeder cells, thereby differentiating into a vascular endothelial cell marker-positive cell population A; and
(II) substantially separating the vascular endothelial cell marker-positive cells from the cell population A obtained in the above step (I).

In the method for production of the present invention, the steps are carried out on the basis of the method for differentiation of the present invention. Therefore, according to the method for production of the present invention, early developmental vascular endothelial cells in which long-term culture and subculture can be performed, the cells can be proliferated *in vitro,* the cells can be subjected to a graft survival at a high efficiency to a primate, for example human and a monkey, preferably a living body of human, and a high compatibility can be obtained, can be produced.

In the method for production of the present invention, the steps are carried out on the basis of the method for differentiation, primate embryonic stem cells, for example, human embryonic stem cells, that can be unlimitedly amplified by self-replication are used. Therefore, according to the method for production of the present invention, the early developmental vascular endothelial cells mentioned above having a substantially uniform quality, and the like can be supplied in a large amount. In addition, according to the method for production of the present invention, since primate embryonic stem cells, especially human embryonic stem cells, are used, a material for the treatment of a condition and/or a disease in which a therapeutic effect can be expected by ameliorating local blood flow can be provided, so that a vascular construction material linking to clinical applications in the field of regenerative therapy can be efficiently produced in a large amount.

In the method for production of the present invention, the steps (I) and (II) are the same as those in the case of the method for differentiation mentioned above.

In the method for production of the present invention, the step of amplifying the cells obtained in the above step (II) {step (III)} may be further carried out. According to the method for production of the present invention, the early developmental vascular endothelial cells mentioned above can be obtained in a larger amount by carrying out the step (III).

In the above step (III), the amplification of the cells obtained in the above step (II) can be carried out by, for example, culturing the cells obtained in the above step (II) on a medium containing an appropriate growth factor, a serum or a serum substitute, or the like in a culture container coated with an extracellular matrix under the conditions appropriate for differentiation until the cells are confluent.

The extracellular matrix mentioned above includes, for example, collagen IV, fibronectin, and the like. The coating of the culture container with the extracellular matrix mentioned above or the like can be carried out by a conventional method.

The growth factor mentioned above includes VEGF (vascular endothelial growth factor), and the like.

The medium which can be used in the above step (III) may be a medium that is appropriate for the amplification of the cells. The medium includes, for example, a medium such as αMEM containing 5 × 10⁻⁵ M 2-mercaptoethanol, a 10% by weight serum, a growth factor, and the like. Concretely, the medium includes, for example, αMEM containing 5 × 10⁻⁵ M 2-mercaptoethanol and a 10% by weight serum, and the like.

The content of the growth factor mentioned above in the medium mentioned above can be appropriately set. From the viewpoint of sufficiently exhibiting the proliferative effects of the cells, in the case of, for example, the VEGF, it is desired that the content of the VEGF in the medium mentioned above is 10 ng/ml or more, and preferably 50 ng/ml or more, and that the content is 100 ng/ml or less, from the viewpoint of sufficiently obtaining the proliferative effects of the early developmental vascular endothelial cells. Within the above range, the concentration can be appropriately set.

The concentration of the serum or serum substitute in the medium mentioned above can be appropriately set. It is desired that the concentration is preferably 2% by weight or more, and that the concentration is 20% by weight or less, and preferably 10% by weight or less. Within the above range, the concentration can be appropriately set.

The culture conditions in the above step (III) include, for example, culturing at 36° to 38°C, and more preferably at 37°C or so (for example, at 37°C) in a vapor phase of 5% by volume CO₂ for 0 to 30 days, and the like.

In the above step (III), at a stage where the vascular endothelial cell marker-positive cells are confluent, the cells are subcultured by the steps of:
- adding a solution appropriate for dissociation of the cells, for example, a 0.25% by weight trypsin solution or the like, in a culture container in which the vascular endothelial cell marker-positive cells are grown;
- removing the vascular endothelial cell marker-positive cells from the culture container; and
- sowing the vascular endothelial cell marker-positive cells obtained on a plate containing the medium ingredients similar to those mentioned above so as to have a cell density of from 1 × 10⁴ cells/cm² to 1.5 × 10⁴ cells, and culturing the cells in the same manner as above,
   whereby the vascular endothelial cell marker-positive cells can be amplified. The subculture can be appropriately repeatedly carried out. In the method for production of the present invention, since primate embryonic stem cells are used, contrary to mouse embryonic stem cells, and the like, unexpectedly, the desired vascular endothelial cell marker-positive cells can be further amplified by the subculture as mentioned above.

In addition, another aspect of the present invention relates to a method for amplifying early developmental vascular endothelial cells, comprising the steps of:
(I) co-culturing primate embryonic stem cells and feeder cells, thereby differentiating into a vascular endothelial cell marker-positive cell population A;
(II) substantially separating the vascular endothelial cell marker-positive cells from the cell population A obtained in the above step (I); and
(III) amplifying the cells obtained in the above step (II).

According to the method for amplification of the present invention, the early developmental vascular endothelial cells can be amplified by the steps similar to those of the method for differentiation and the method for production mentioned above. In addition, in the method for amplification of the present invention, primate embryonic stem cells, for example, human embryonic stem cells, that can be unlimitedly amplified by self-replication are used. Therefore, the early developmental vascular endothelial cells mentioned above having a substantially uniform quality can be supplied in a large amount.

The early developmental vascular endothelial cells obtained by the method for differentiation, the method for production, and the method for amplification of the present invention have a substantially uniform quality and can be supplied in a large amount; therefore, according to the method for differentiation, the method for production, and the method for amplification of the present invention, a material for revascularization (for example, treatment of a lesion site of a blood vessel, or the like), or the like can be stably supplied. The early developmental vascular endothelial cells obtainable by the present invention have excellent characteristics that the regeneration of a blood vessel or the like can be performed, that the cells can be subjected to a graft survival at a high efficiency, and that the cells show especially a high compatibility to a primate, concretely human and a monkey, especially a living body of human.

Therefore, another aspect of the present invention relates to early developmental vascular endothelial cells obtainable by differentiating primate embryonic stem cells into a vascular endothelial cell marker-positive cell population.

Since the early developmental vascular endothelial cells of the present invention are produced from primate embryonic stem cells, the cells can be subjected to a graft survival at a high efficiency, and can be used in a high compatibility with an individual.

The early developmental vascular endothelial cells of the present invention have an ability of differentiating into vascular endothelial cells of a primate, and show a graft survival at a high efficiency and a high compatibility with an individual. Therefore, according to the early developmental vascular endothelial cells of the present invention, vascular endothelial cells can be developed in an individual, so that the cells enable the treatment of a condition and/or a disease in which a therapeutic effect can be expected by ameliorating local blood flow.

The graft survival efficiency and the compatibility mentioned above can be evaluated by, for example, injecting cells to be tested to a skin ulcer site of a nude mouse, and specifically staining the vascular endothelia via the presence of a marker specific to the cells to be tested mentioned above in the skin ulcer site, and further via blood flow of an individual, whereby the construction of a functional blood vessel communicating with blood flow of an individual is confirmed.

The therapeutic effects by revascularization can be evaluated by, for example, injecting cells to be tested to a skin ulcer site of a nude mouse, observing the skin lesion site with the passage of time, and observing a change in the size of the skin ulcer site by the injection of the cells.

The early developmental vascular endothelial cells of the present invention can be evaluated as the desired early developmental vascular endothelial cells by using as an index that shows positive by immunostaining or the like using an antibody against the vascular endothelial cell marker that is different from the antigen of the antibody used in sorting, namely that that shows positive for any of VE-cadherin, CD34, PECAM1, and VEGF-R2 are positive, or the like.

The early developmental vascular endothelial cells of the present invention can be stably stored by, for example, lyophilizing the cells in liquid nitrogen in the presence of 10% by weight dimethyl sulfoxide and 90% by weight serum.

In addition, according to the early developmental vascular endothelial cells of the present invention, a vascular lesion-therapeutic agent can be provided.

Therefore, a still another aspect of the present invention relates to a vascular lesion-therapeutic agent, containing early developmental vascular endothelial cells obtainable by differentiating primate embryonic stem cells into a vascular endothelial cell marker-positive cell population as an active ingredient.

The vascular lesion-therapeutic agent of the present invention can also be used in the treatment of a disease such as an ischemic disease, for example, arteriosclerosis obliterans, an ischemic heart disease (such as myocardial infraction, angina pectoris or heart failure), a cerebrovascular disease, skin ulcer, ischemic enteritis or nephrosclerosis, regeneration of a blood vessel in a wounded site, or the like.

The phrase "site in need of regeneration of a blood vessel" mentioned above as used herein includes, for example, a vascular lesion site, and the like. More concretely, the "site in need of regeneration of a blood vessel" mentioned above includes, for example, an ischemic site such as a lower limb ischemic site, a myocardial ischemic site and a cerebral ischemic site, a skin ulcer site, and portion where a blood vessel is cut or broken in a wounded site, and the like.

The vascular lesion-therapeutic agent of the present invention may appropriately contain a solution suitable for maintaining the early developmental vascular endothelial cells obtained by the present invention (for example, a pharmaceutically acceptable buffer or the like), a pharmaceutically acceptable adjuvant, a substance capable of promoting angiogenesis, a sustained-release carrier containing the substance, or the like. The substance capable of promoting angiogenesis mentioned above includes bFGF, VEGF, HGF, and a substance which allows to exhibit a function equivalent to that of these substances, and the like. In addition, the sustained-release carrier mentioned above includes gelatin particles, carriers which allow to exhibit a function equivalent to that of the gelatin particles, and the like.

The content of the early developmental vascular endothelial cells in the vascular lesion-therapeutic agent of the present invention may be within the range suitable for maintaining viability and functions of the early developmental vascular endothelial cells, maintaining an ability of differentiating the early developmental vascular endothelial cells into other vascular endothelial cells, or the like.

The vascular lesion-therapeutic agent of the present invention can be directly or indirectly administered to a site in need of regeneration of a blood vessel or the like, depending on the disease to which the therapeutic agent is to be applied, severity of the disease, age of an individual to which the therapeutic agent is to be administered, a physical strength of the individual, a site of the disease, or the like. The method for administering the vascular lesion-therapeutic agent of the present invention concretely includes, for example, local injection, intravascular administration, delivery via a catheter and the like. In addition, upon administering the vascular lesion-therapeutic agent of the present invention, a surgical operation or the like may be performed, if necessary.

The dose of the vascular lesion-therapeutic agent of the present invention may be a "therapeutically effective amount," that is, an amount suitable for carrying out a graft survival at the site to which the therapeutic agent is to be applied, depending on the disease to which the therapeutic agent is to be applied, severity of the disease, age of an individual to which the therapeutic agent is to be administered, a physical strength of the individual, a site of the disease or the like, which may be an amount by which a regenerative effect of a blood vessel (for example, effect of treating vascular lesion) can be sufficiently exhibited.

The effect of the vascular lesion-therapeutic agent of the present invention can be examined by, for example, observing a site in need of regeneration of a blood vessel by a means of angiography, immunostaining of a blood vessel tissue of a tissue section or the like, assaying an amount of blood flow by a laser Doppler method, quantifying a lesion of myocardial infraction or cerebral infraction, observing movements of the heart muscles by means of cerebral blood-flow scintigraphy, echo or the like, in which the regeneration of a vascular structure, amelioration in blood flow, amelioration in functions in an ischemic site, amelioration in local blood pressure, normalization of a skin temperature, augmentation of capillary blood vessels in a site in need of regeneration of a blood vessel (for example, a site of vascular lesion or the like) or the like caused by administration of the vascular lesion-therapeutic agent of the present invention as compared to that before the administration is used as an index for regeneration of a blood vessel.

According to the early developmental vascular endothelial cells of the present invention, revascularization can be carried out.

Therefore, other aspect of the present invention relates to a method for revascularization, characterized in that the method comprises supplying early developmental vascular endothelial cells obtained by differentiating primate embryonic stem cells into a vascular endothelial cell marker-positive cell population to a site in need of regeneration of a blood vessel, for example, a site of vascular lesion or the like.

In the method for revascularization of the present invention, in one embodiment, early developmental vascular endothelial cells obtainable by the method for production of the present invention are used.

In the method for revascularization of the present invention, the early developmental vascular endothelial cells mentioned above are used; therefore, according to the method for revascularization of the present invention, a lesion site of a blood vessel can be treated, and a condition and/or a disease in which a therapeutic effect can be expected by ameliorating local blood flow or the like can be treated. In addition, in the method for revascularization of the present invention, the early developmental vascular endothelial cells mentioned above are used; therefore, according to the method for revascularization of the present invention, a blood vessel can be regenerated in a site in need of the regeneration of a blood vessel.

The method for revascularization of the present invention can be carried out by directly or indirectly administering the early developmental vascular endothelial cells mentioned above to a site to be treated, in the same manner as in the method for administering the vascular lesion-therapeutic agent of the present invention. The method for administering the early developmental vascular endothelial cells mentioned above to a site to be treated concretely includes, for example, a local injection, an intravascular administration, a delivery via a catheter and the like. Here, upon administration of the early developmental vascular endothelial cells mentioned above to a subject site, a surgical operation or the like may be performed, if necessary.

The dose of the early developmental vascular endothelial cells of the present invention can be set in the same manner as in the case of the vascular lesion-therapeutic therapeutic agent mentioned above, and may be an amount suitable for performing graft survival at the site to which the cells are to be applied, which may be an amount by which a regenerative effect of a blood vessel can be sufficiently exhibited.

In addition, the regenerative effect by the method for revascularization of the present invention can be evaluated by the same techniques and indexes as those in the evaluation of the effect of the vascular lesion-therapeutic agent mentioned above. Concretely, the regenerative effect mentioned above can be evaluated, for example, by observing the subject site by angiography, immunostaining of a tissue section of a blood vessel tissue or the like, assaying an amount of blood flow by laser Doppler method or the like, in which regeneration of a blood vessel structure, amelioration in blood flow, amelioration in functions in a subject site, amelioration in local blood pressure, normalization of a skin temperature, or augmentation of capillary blood vessels in a site to be treated or the like, caused by the administration of the early developmental vascular endothelial cells mentioned above as compared to that before the administration is used as an index of regeneration of a blood vessel.

In the method for revascularization of the present invention, a substance capable of promoting angiogenesis, a sustained-release carrier containing the substance, or the like may be further administered after the administration of the early developmental vascular endothelial cells mentioned above.

In addition, in the method for revascularization of the present invention, the early developmental vascular endothelial cells mentioned above may be used in co-existence with mural cells or cells serving as their precursor cells in terms of differentiation lineage.

In addition, in one aspect, the present invention relates to a method for transplantation, characterized in that the method comprises transplanting the early developmental vascular endothelial cells obtained by the method for production of the present invention into a living body.

The method for transplantation of the present invention can be carried out in the same manner as in the method for revascularization of the present invention. In the method for transplantation of the present invention, the early developmental vascular endothelial cells obtainable by the method for production of the present invention are used; therefore, according to the method for transplantation of the present invention, a graft survival at a high efficiency in a living body and a high compatibility to an individual can be obtained. In addition, in the method for transplantation of the present invention, the early developmental vascular endothelial cells obtainable by the method for production of the present invention are used; therefore, according to the method for transplantation of the present invention, a blood vessel can be regenerated at a site into which the cells are to be transplanted in an individual. Therefore, the method for transplantation of the present invention enables the treatment of a disease in which a therapeutic effect can be expected by regenerating a blood vessel, for example, the treatment of the ischemic disease mentioned above or the like, recovery of a blood vessel in a wound, or the like.

The method for transplantation of the present invention can be carried out by a process comprising, for example, the steps of:
1) specifying a site of a disease, a site in need of transplantation, a site in which a therapeutic effect can be expected by transplantation, or the like in an individual in need of the transplantation of the early developmental vascular endothelial cells obtainable by the method for production of the present invention;
2) carrying out a treatment suitable for transplantation of the early developmental vascular endothelial cells obtainable by the method for production of the present invention to the site specified in the above step 1) (the site at which the cells are to be transplanted); and
3) transplanting the early developmental vascular endothelial cells obtained by the method for production of the present invention into the site at which the cells are to be transplanted in the individual after the above step 2). In addition, after the above step 3), an agent capable of promoting angiogenesis or the like may be appropriately administered to an individual after the transplantation.

The identification of the "site into which the cells are to be transplanted" in the above step 1) can be carried out by, for example, identifying the pathogenesis and specifying a site of the disease by means of angiography, diagnosis by X-ray irradiation, CT (computed tomography) scan, MRI (magnetic resonance imaging), expression of various kinds of diagnostic markers, echoangiography, scintigraphy, ABI determination, percutaneous enzyme-localization determination, or the like.

The site at which the cells are to be transplanted includes, for example, a site similar to the "site in need of regeneration of a blood vessel" mentioned above.

The "treatment suitable for transplantation of the early developmental vascular endothelial cells" in the above step 2) includes an exposure of the site mentioned above into which the cells are to be transplanted by means of a surgical operation for facilitating a delivery of the early developmental vascular endothelial cells to the site mentioned above into which the cells are to be transplanted; an insertion of a catheter for facilitating a delivery of the early developmental vascular endothelial cells to the site mentioned above into which the cells are to be transplanted; a treatment of the early developmental vascular endothelial cells for delivery of the early developmental vascular endothelial cells to the site mentioned above into which the cells are to be transplanted in a stable state; and the like.

In the above step 3), the transplantation of the early developmental vascular endothelial cells can be carried out by local injection, intravascular administration, a delivery via a catheter, or the like.

The amount of the early developmental vascular endothelial cells usable during the transplantation in the above step 3) can be set in the same manner as in the case of the vascular lesion-therapeutic agent mentioned above, and may be suitable for performing a graft survival into a site at which the cells are to be transplanted, which may be an amount by which a regenerative effect of a blood vessel or the like can be sufficiently exhibited.

The method for transplantation of the present invention can be applied to a primate, for example, a human, a monkey or the like.

In addition, the method for transplantation of the present invention can be applied to a treatment of a disease such as an ischemic disease, for example, arteriosclerosis obliterans, ischemic heart disease (such as myocardial infraction, angina pectoris, or heart failure), cerebrovascular disorder, skin ulcer or the like, regeneration of a blood vessel at a lesion site, or the like.

A still another aspect of the present invention relates to a method for treating a condition and/or a disease in which a therapeutic effect can be expected by ameliorating local blood flow in a mammal to be treated, characterized in that the method comprises administering a therapeutically effective amount of the early developmental vascular endothelial cells obtained by the method for production of the present invention to a mammal to be treated having a condition and/or a disease in which a therapeutic effect can be expected by ameliorating local blood flow. The method for treatment of the present invention is effective for a condition and/or a disease in which a therapeutic effect can be expected by ameliorating local blood flow, for example, ulcer, an ischemic disease or the like.

The method for treatment of the present invention can be carried out by using the vascular lesion-therapeutic agent of the present invention, carrying out the method for transplantation of the present invention, or the like.

Furthermore, the early developmental vascular endothelial cells of the present invention can be used in the manufacture of a medicament for treating a mammal to be tested by supplying the early developmental vascular endothelial cells to a site in need of regeneration of a blood vessel in a mammal to be tested, and regenerating a blood vessel, for example, a vascular lesion site, or the like, wherein the mammal to be tested has a condition and/or a disease in which a therapeutic effect can be expected by ameliorating local blood flow.

Therefore, another aspect of the present invention relates to use of the early developmental vascular endothelial cells obtained by differentiating primate embryonic stem cells into a vascular endothelial cell marker-positive cell population, in the manufacture of a medicament for treating a mammal to be tested by supplying the early developmental vascular endothelial cells to a site in need of regeneration of a blood vessel of a mammal to be tested, and revascularizing the site, wherein the mammal to be tested has a condition and/or a disease in which a therapeutic effect can be expected by ameliorating local blood flow.

The early developmental vascular endothelial cells of the present invention have more excellent supplying ability and exhibit a higher graft survival, as compared to those of the progenitor cells of the vascular endothelial cells.

In addition, a vascular structure including mature vascular endothelial cells can be generated by culturing the early developmental vascular endothelial cells of the present invention in an environment suitable for angiogenesis.

Therefore, a still other aspect of the present invention is a method for constructing a vascular structure, characterized in that the method comprises culturing the early developmental vascular endothelial cells obtainable by differentiating primate embryonic stem cells in an environment suitable for angiogenesis.

The method for constructing a vascular structure of the present invention includes a method comprising the step of
(III') culturing the above vascular endothelial cell marker-positive cells in an environment suitable for angiogenesis or a blood vessel environment,
in addition to the steps (I) and (II), and optionally the step (III), of the method for producing early developmental vascular endothelial cells mentioned above.

The method for constructing a vascular structure of the present invention enables stable provision of a material for treatment of a condition and/or a disease in which a therapeutic effect can be expected by improving local blood flow, or the like. In addition, in the method for constructing a vascular structure of the present invention, the early-developmental vascular endothelial cells (vascular endothelial cell marker-positive cell population) obtained by differentiating a primate embryonic stem cell are used; therefore, according to the method for constructing a vascular structure of the present invention, there can be provided a material for treatment of the condition and/or disease mentioned above or the like in which the cells are subjected to a graft survival at a high efficiency, a vascular structure can be constructed, and a high compatibility to an individual can be obtained.

The phrase "environment or blood vessel environment suitable for angiogenesis" includes, in the case of *ex vivo* conditions, for example, conditions of carrying out a three-dimensional culture of the early developmental vascular endothelial cells mentioned above is carried out within a carrier made of a collagen gel (for example, collagen IA gel or the like), a matrigel, or the like at 37°C in the presence of 5% by volume of CO₂ in an appropriate culture medium (for example, α-MEM containing 5 × 10⁻⁵ M 2-mercaptoethanol and 10% by weight serum) containing VEGF and phorbol 12-myristate 13-acetate, and the like. The concentration of VEGF in the culture medium is similar to those mentioned above. In addition, from the viewpoint of obtaining appropriate formation of a tubing structure, it is desired that the concentration of the phorbol 12-myristate 13-acetate in the culture medium is from 10 to 100 µM, and more preferably 100 µM. In addition, the phrase "environment or blood vessel environment suitable for angiogenesis" mentioned above includes, in the case of *in vivo* conditions, conditions of maintaining the early-developmental vascular endothelial cells mentioned above within a blood vessel or in the surroundings of the blood vessel, and the like.

According to the method for constructing a vascular structure of the present invention, a vascular structure can be constructed.

Therefore, in the method for constructing a vascular structure of the present invention, other vascular cells such as mural cells may be used in coexistence, in addition to the early developmental vascular endothelial cells, in the above step (III').

The present invention will be specifically described hereinbelow by the Examples, without intending to limit the scope of the present invention to these Examples and the like.

### EXAMPLE 1

### (1) Differentiation from Monkey Embryonic Stem Cells

As monkey embryonic stem cells, CMK-6 cell strain, which is cynomolgus monkey (*Macaca fascicularis*) embryonic stem cells, [Hirofumi Suemori et al., Developmental Dynamics, 222, 273-279 (2001)] was used.

A cell dissociation buffer {manufactured by GIBCO} was added to the monkey embryonic stem cells maintained on a dish containing a medium for maintaining undifferentiated cells {composition per 200 ml: 163 ml of Dulbecco's modified minimum essential medium (DMEM)/F12, 30 ml of fetal bovine serum (final concentration: 15% by weight), 2 ml of L-glutamine (final concentration: 2 mM), 2 ml of penicillin (100 U/ml)-streptomycin (100 µg/ml), 2 ml of MEM non-essential amino acid solution (manufactured by GIBCO), 1 ml of 2-mercaptoethanol (final concentration: 0.1 mM)}. The monkey embryonic stem cells were incubated at 37°C for 10 minutes. Thereafter, the embryonic stem cells were collected by tapping the dish and then detaching the cells in the state of a single cell from the dish mentioned above by pipetting.

OP9 cell strain was cultured to be confluent on a 10-cm dish coated with 0.1% by weight of an aqueous gelatin solution {derived from bovine skin, manufactured by SIGMA} to obtain an OP9 feeder layer.

The collected embryonic stem cells mentioned above were sown on the OP9 cell strain mentioned above at a cell density of 5 × 10⁴ cells. Thereafter, 20 ml of a differentiation culture medium {composition: αMEM culture medium containing 5 × 10⁻⁵ M 2-mercaptoethanol and 10% by weight serum (manufactured by GIBCO)} was added to the cells. The embryonic stem cells were co-cultured with OP9 cells at 37°C in 5% by volume of CO₂ for 8 to 10 days.

The culture supernatant was removed from the resulting culture, and the dish was washed with a phosphate-buffered physiological saline. Next, 2 ml of the cell dissociation buffer was added to the dish after washing. The cells were incubated at 37°C for 10 minutes. As a result, the OP9 cells remained in a sheet-like structure, and embryonic stem cells-derived cells were detached in the state of a single cell. Subsequently, a solution on a dish obtained after the incubation mentioned above was passed through a cell strainer (filter having a diameter of 70 µm) {manufactured by BD Biosciences}, thereby collecting only the embryonic stem cells-derived cell population.

Next, 1 × 10⁶ cells of the resulting cell population were suspended per 100 µl of a HBSS solution {manufactured by GIBCO} containing 1% by weight bovine serum albumin (BSA) {manufactured by SIGMA}. To the resulting cell suspension, an anti-VE cadherin antibody {manufactured by BD Biosciences} was added and incubated at room temperature for 20 minutes. Thereafter, the resulting product was washed twice with a HBSS solution containing 1% by weight BSA, and subjected to flow cytometry analysis. In the flow cytometry analysis mentioned above, FACSVantage {manufactured by BD Biosciences} was used.

As a result, in the resulting embryonic stem cells-derived cell population, a cell population expressing VE cadherin, which is a vascular endothelial cell marker, was found.

In addition, the VE cadherin-positive cells mentioned above were CD34-positive and PECAM1-positive and VEGF-R2-positive according to the flow cytometry analysis; therefore, it was suggested that the VE cadherin-positive cells are vascular endothelial cells.

Next, the VE cadherin-positive cell population were separated from the embryonic stem cell-derived cell population mentioned above by means of cell sorting according to the trade name: FACSVantage {manufactured by BD Biosciences}, using the anti-VE cadherin antibody mentioned above.

### (2) Differentiation from Human Embryonic Stem Cells

As human embryonic stem cells, HES-3 cell strain established in Australia Monash University { Benjamin E. Reubinof et al., Nature Biotechnology, 18, 399-404 (2000)} was used.

Human embryonic stem cells were treated at 37°C for 10 minutes using 0.1 % by weight collagenase (manufactured by Wako Pure Chemical Industries, Ltd.). Undifferentiated human embryonic stem cells were collected in the form of a small cluster by tapping and pipetting. Next, the resulting human embryonic stem cells were sown on an OP9 feeder layer of a gelatin-coated dish in the same manner as in the monkey embryonic stem cells of Example 1. The human embryonic stem cells in the form of small clusters were co-cultured with the OP9 cells.

Thereafter, VE cadherin-positive cells appeared on the eighth to tenth days from the beginning of the co-culture.

Subsequently, the VE cadherin cells were subjected to flow cytometry using a vascular endothelial cell marker in the same manner as in the above (1).

As a result, the VE cadherin-positive cells were CD34-positive and VEGF-R2-positive and PECAM1-positive according to the flow cytometry analysis; therefore, it was suggested that the VE cadherin-positive cells are vascular endothelial cells.

### EXAMPLE 2

The VE cadherin-positive cell population obtained in the above item (2) of Example 1 (5 × 10⁴ cells) were sown in each well of a 6-well dish {manufactured by BD Biosciences} coated with collagen IV or fibronectin, and 20 ml of a culture medium {α-MEM culture medium (manufactured by GIBCO) containing 5 × 10⁻⁵ M 2-mercaptoethanol and 10% by weight serum) in the presence of 50 ng/ml human recombinant VEGF (trade name) {manufactured by PEPROTECH} was added thereto. The cells were cultured at 37°C in the presence of 5% by volume of CO₂. Here, the culture medium mentioned above was exchanged once in every two days.

At a stage where the VE cadherin-positive cells reached confluence, the VE cadherin-positive cells were detached from the dish with a 0.25% by weight trypsin solution {manufactured by GIBCO}. The resulting cells were diluted with the culture medium mentioned above so as to have a dilution fold of 1:2 to 1:3, and the resulting cells were further sown in each well of a 6-well dish {manufactured by BD Biosciences} coated with collagen IV or fibronectin, in the same manner as above. The cells were cultured, and then subjected to subculture using a fresh culture medium in the presence of 50 ng/ml human recombinant VEGF (trade name) {manufactured by PEPROTECH}.

The resulting cells were subjected to flow cytometry analysis in the same manner as in Example 1 mentioned above.

As a result, about 35% of the resulting cells turned out to be VE cadherin-positive, CD34 positive, VEGF-R2 positive, PECAM 1 positive, even when the cells were subjected to 6th subculture. Therefore, about 35% of the resulting cells are VE cadherin-positive cells having the same characteristics as the cells before the culture, suggesting that the cells are early developmental vascular endothelial cells.

### COMPARATIVE EXAMPLE 1

The same procedures as in Examples 1 and 2 mentioned above were carried out using mouse embryonic stem cells.

As a result, when the mouse embryonic stem cells were used, VE cadherin-positive cells could not be proliferated, because the cells died during the course of subculture under the same procedures as in Example 2 mentioned above.

### EXAMPLE 3

Transplantation of Vascular Endothelial Marker-Positive Cells into Skin Ulcer Model

Skin ulcer was generated on both sides of back side of 8- to 11-week-old KSN nude mice (Japan SLC, Inc.) with a product under the trade name of KAI STERILE DERMAL BIOPSY PUNCH (manufactured by kai industries co., ltd., size: 4.00 mm, Commercial Product No. BP-40F).

In addition, the VE cadherin-positive cells proliferated by subculture obtained in Example 2 (5 × 10⁵ cells/50 µl phosphate buffered saline) was labeled with a product under the trade name of Vybrant CM-DiI cell-labeling solution {manufactured by Molecular Probes}, in accordance with the manual of Molecular Probes attached to the manufactured article.

The labeled VE cadherin-positive cells were subcutaneously injected to a skin ulcer on one side of the KSN nude mice mentioned above. In addition, as a control, only 50 µl of phosphate buffered saline (manufactured by GIBCO) was injected on the opposite side of the skin ulcer to which the VE cadherin-positive cells were injected, whereby the cells were transplanted, and curing process of the skin ulcer was observed.

As a result, it can be seen that no difference in area of the skin ulcers is found on the both sides three days after the injection of cells as shown in Panel (A) of Figure 1 and Panel (A) of Figure 2, whereas the area of the skin ulcer is significantly reduced on the side to which the proliferated VE cadherin-positive cells were subcutaneously injected six days after the injection as shown in Panel (B) of Figure 1 and Panel (B) of Figure 2.

In addition, the KSN mice mentioned above was subjected to intraperitoneal injection of 10 µl of 16% Nembutal (registered trademark, manufactured by Dainippon Pharmaceutical Co., Ltd.) per gram of body weight seven days after transplantation of the cells to anesthetize the KSN nude mice.

Next, 100 µl of a product under the trade name of FLUORESCEIN GRIFFONIA SIMPLICIFOLIA LECTIN I, ISOLECTIN B4 {manufactured by VECTOR} was intravenously injected via the portal vein. Thereafter, 4% by weight paraformaldehyde (manufactured by Muto Kagaku) was intravenously injected via the portal vein by dripping for 30 minutes. The mice were subjected to reflux fixation, and a skin tissue was then excised, and subjected to then to freeze fixation, to prepare tissue sections.

The resulting tissue sections were observed under a fluorescent microscope (manufactured by Carl Zeiss, trade name: LSM-5 PASCAL). The results are shown in Figure 3.

As a result, as shown in Figure 3, cells which are found to be ISOLECTIN B4-positive and DiI-positive under the skin on the side to which VE cadherin-positive cells were subcutaneously injected, showing that the subcutaneously injected VE cadherin-positive cells are incorporated into a blood vessel of the KSN nude mice.

### EXAMPLE 4

Transplantation of Vascular Endothelial Marker-Positive Cells into Lower-Limb Ischemic Model

Pentobarbital (manufactured by Dainippon Pharmaceutical Co., Ltd.) was intraperitoneally administered to 8-week-old KSN male nude mice (Japan SLC, Inc.) at 80 mg/kg, to anesthetize the KSN nude mice.

Thereafter, skin of the right femoral region was vertically incised, to expose femoral arteries and veins. The arteries and veins were detached from each other, and the femoral veins were ligated. The resulting mice were used as a lower-limb ischemic model mouse.

In addition, the VE cadherin-positive cells (1 × 10⁶ cells) obtained in Example 2 were labeled with a product under the trade name of Vybrant CM-DiI cell-labeling solution (manufactured by Molecular Probes), to prepare a cell suspension with 100 µl of phosphate buffered saline in the same manner as described above.

The cell suspension mentioned above was intraarterially injected to the KSN nude mice mentioned above via the right femoral artery using an insulin syringe equipped with a 29G needle (manufactured by TERUMO CORPORATION). Immediately after the injection, the above arteries were ligated and excised. These procedures are schematically shown in Figure 4. Here, as a control group, only the phosphate buffered saline without containing any cells was intraarterially injected in the same manner as in the case of the cell suspension mentioned above. The results are shown in Figure 5.

After the transplantation of the VE cadherin-positive cells, blood flow of the skin surface of the ischemic lateral crural muscle was observed with the passage of time using laser Doppler blood flowmeter {manufactured by Moor} on each of the 0th, 14th, 28th and 42nd days.

As a result, in the group into which the cells were transplanted, significant recovery of blood flow is found in the ischemic lateral crural muscles as compared to the control group. In addition, as shown in Figure 5, when the blood flow of the skin surface of the ischemic lateral crural muscle is observed with the passage of time, significant recovery in blood flow is found was observed in the group into which the cells are transplanted as compared to the control group.

The cell suspension mentioned above was intraarterially injected to via the right femoral arteries of the KSN nude mice, and the above arteries were ligated and excised immediately after the injection, in the same manner as described above. Here, the resulting mice were used as a lower-limb ischemic model mouse.

Pentobarbital was intraperitoneally administered to the lower-limb ischemic model mouse mentioned above at 80 mg/kg seven days after transplantation of the cell population, to anesthetize the KSN nude mice (lower-limb ischemic model mouse).

Next, a thoracic cavity of the anesthetized KSN mice (lower-limb ischemic model mouse) was incised. A 23G injection needle (TERUMO CORPORATION) was inserted to the left ventricle, and the right ventricle was opened. Thereafter, perfusion was carried out with physiological saline for 15 minutes, and further perfusion was carried out with phosphate buffered saline containing 4% by weight paraformaldehyde for 15 minutes to be immobilized. The ischemic lateral crural muscles were then collected, to prepare a tissue specimen.

The resulting tissue specimen was observed under a fluorescence stereoscopic microscope (manufactured by Leica). The results are shown in Figure 6.

As a result, as shown in Figure 6, it is shown that labeled transplanted cells are subjected to a graft survival along a vessel in a wide range in the tissue specimen of the ischemic lateral crural muscles seven days after the transplantation of the VE cadherin-positive cells. Therefore, it can be seen that the transplanted VE cadherin-positive cells were incorporated into the blood vessel of the KSN nude mice (lower-limb ischemic model mouse).

In addition, on the 42nd day after the transplantation of the VE cadherin-positive cells, an anesthetized KSN nude mice (lower-limb ischemic model mouse) was immobilized with paraformaldehyde-containing phosphate buffered saline in the same manner as above, and the ischemic lateral crural muscles were then collected. Solution components in the tissue after the immobilization were exchanged with 30% by weight of sucrose-containing phosphate buffer (pH 7.2). Thereafter, the tissue mentioned above was embedded and frozen in a product under the trade name of OCT compound (manufactured by Sakura Seiki Kabushiki Kaisha), to prepare tissue sections with a cryostat (manufactured by Leica). As a control group, tissue sections were prepared in the same manner, for a KSN nude mice (lower-limb ischemic model mouse) into which phosphate buffered physiological saline was injected without transplanting the VE cadherin-positive cells.

The resulting tissue sections were subjected to immunostaining with an anti-mouse PECAM 1 antibody and an anti-human PECAM 1 antibody. The results are shown in Figure 7.

As a result, as shown in Panel (B) of Figure 7, in the site into which the cells were transplanted in the ischemic lateral crural muscle on the 42nd day after the transplantation of the VE cadherin-positive cells, anti-human PECAM 1 antibody-positive cells were detected. Therefore, it can be seen that human vascular endothelial cells derived from the transplanted VE cadherin-positive cells are present.

In addition, the KSN nude mice (lower-limb ischemic model mouse) mentioned above into which the VE cadherin-positive cells mentioned above were transplanted was injected with 100 µl of a product under the trade name of GRIFFONIA SIMPLIFOLIA LECTIN I, ISOLECTIN B4 {manufactured by VECTOR} via a portal vein immediately before analgesic sacrifice. After 15 minutes, the mice were subjected to reflux fixation in the same manner as above, and then to freeze embedment to prepare tissue sections.

The resulting tissue sections were subjected to immunostaining with an anti-human PECAM 1 antibody. The results are shown in Figure 8.

As a result, as shown in Figure 8, cells which are ISOLECTIN B4-positive and human vascular endothelial marker-positive were detected. Therefore, it can be seen that the transplanted VE cadherin-positive cells are incorporated into the blood vessel of the KSN nude mice.

### INDUSTRIAL APPLICABILITY

The present invention enables application to a treatment of a condition or a disease in which a therapeutic effect can be expected by improving local blood flow.

## Claims

1. A method for differentiating primate embryonic stem cells into early developmental vascular endothelial cells, **characterized in that** the method comprises differentiating primate embryonic stem cells into vascular endothelial cell marker-positive cell population.

2. The method according to claim 1, wherein the method comprises the steps of:
(I) co-culturing primate embryonic stem cells and feeder cells, thereby differentiating into a cell population A comprising vascular endothelial cell marker-positive cells; and
(II) substantially separating the vascular endothelial cell marker-positive cells from the cell population A obtained in said step (I).

3. A method for producing early developmental vascular endothelial cells, comprising the steps of:
(I) co-culturing primate embryonic stem cells and feeder cells, thereby differentiating into a cell population A comprising vascular endothelial cell marker-positive cells; and
(II) substantially separating the vascular endothelial cell marker-positive cells from the cell population A obtained in said step (I).

4. The method according to claim 3, further comprising the step of (III) amplifying the cells obtained in said step (II).

5. A method for amplifying early developmental vascular endothelial cells, comprising the steps of:
(I) co-culturing primate embryonic stem cells and feeder cells, thereby differentiating into a cell population A comprising vascular endothelial cell marker-positive cells;
(II) substantially separating the vascular endothelial cell marker-positive cells from the cell population A obtained in said step (I); and
(III) amplifying the cells obtained in said step (II).

6. Early developmental vascular endothelial cells, obtainable by the method as defined in claim 3 or 4.

7. A vascular lesion-therapeutic agent, comprising early developmental vascular endothelial cells obtainable by differentiating primate embryonic stem cells as an active ingredient.

8. A method for revascularization, **characterized in that** the method comprises supplying early developmental vascular endothelial cells obtainable by differentiating primate embryonic stem cells to a site in need of regeneration of a blood vessel.

9. A method for revascularization, **characterized in that** the method comprises supplying early developmental vascular endothelial cells obtainable by the method as defined in claim 3 or 4 to a site in need of regeneration of a blood vessel.

10. A method for transplantation, **characterized in that** the method comprises transplanting early developmental vascular endothelial cells obtainable by the method as defined in claim 3 or 4 into a living body.

11. A method for treating a condition or a disease in which a therapeutic effect can be expected by ameliorating local blood flow in a mammal individual to be tested, **characterized in that** the method comprises administering early developmental vascular endothelial cells obtainable by the method as defined in claim 3 or 4 in a therapeutically effective dose to the mammal individual to be tested, wherein the mammal individual to be tested has a condition or a disease in which a therapeutic effect can be expected by ameliorating local blood flow.

12. Use of early developmental vascular endothelial cells obtainable by differentiating primate embryonic stem cells in the manufacture of a medicament for treating a mammal individual to be tested by supplying to a site in need of regeneration of a blood vessel in the mammal individual to be tested, and revascularizing the site, the mammal individual having a condition and/or a disease, in which a therapeutic effect can be expected by ameliorating local blood flow.

13. A method for constructing a vascular structure, **characterized in that** the method comprises culturing early developmental vascular endothelial cells obtainable by differentiating primate embryonic stem cells in an environment appropriate for angiogenesis.

14. The method according to claim 13, comprising the steps of:
(I) co-culturing primate embryonic stem cells and feeder cells, thereby differentiating into a cell population A comprising vascular endothelial cell marker-positive cells;
(II) substantially separating the vascular endothelial cell marker-positive cells from the cell population A obtained in the above step (I); and
(III') culturing the above vascular endothelial cell marker-positive cells in an environment appropriate for angiogenesis, or a blood vessel environment.

15. The method according to claim 14, further comprising the step, prior to the step (III'), of (III) amplifying the cells obtained in said step (II).
